# EUROPEAN PATENT APPLICATION

(11) **EP 1 333 096 A1**
(43) Date of publication of application: **06.08.2003**
(21) Application number: 03005685.7
(22) Date of filing: 01.09.2000
(51) Int. Cl.: C12Q 1/00, C12Q 1/70, C12Q 1/68, G01N 33/53, G01N 33/543, C12N 7/00, C12N 15/00, C12N 15/09, C12N 15/63, C12N 15/70, C12N 15/10

(54) **METHOD TO IDENTIFY GENE FUNCTION USING PHAGE DISPLAY SCREENING**

(62) Divisional of application: 00961492.6
(71) Applicant: Yale University, New Haven, CT 06520-8107 (US)
(72) Inventor: Austin, David J., New Haven, Connecticut 06250 (US); Savinov, Sergey N., Philadelphia, PA 19143 (US); Sche, Paul P., New Haven, Connecticut 06511 (US); McKenzie, Kathleen M., New Haven, Connecticut 06511 (US)
(74) Representative: Gates, Marie Christina Esther

(57) **Abstract**

Methods for identifying genes which bind to natural products and other small molecules using a phage display system are described. Phage displaying protein which binds the target molecule are selected by affinity chromatography using a solid support to which a small molecule non-peptide bait is coupled. The selectively bound phage can then be amplified and the DNA inserts encoding the binding protein sequenced. The specifically bound protein is then useful to screen for therapeutic candidates which can then be used to treat conditions modulated by the target protein.

## Description

### Technical Field

The invention relates to gene discovery, in particular, identification of gene function. The invention concerns the use of phage display techniques to monitor ligand/receptor interactions where the ligand is a typical small molecule pharmaceutical.

### Background Art

Numerous methods to detect the interaction of ligands with their receptors have been described. Most drug discovery endeavors appear to focus on high throughput screening of libraries of compounds which are potential agonists or antagonists against a cloned receptor known to be a pharmaceutically important target. The converse endeavor, *i.e.* determination of the natural receptor for a pharmaceutical or other small molecule such as a natural product, has been less reported. Such an approach is, however, of great significance for example, because identification of ligand/receptor binding for a candidate pharmaceutical would elucidate not only the mechanism of action of the pharmaceutical, but would also identify pathways by which unwanted adverse reactions might occur, and additional mechanisms of action.

Previous methods of investigating small molecule - protein interactions have involved the use of whole cells, which have inherent limitations. These methods include the use of stochastic nanodroplets as described by Borchardt, A., *et al., Chem. Biol.* (1997) 4:961-968; and by Huang, J., *et al., Proc. Natl. Acad. Sci. USA* (1997) 94:13396-13401; the use of a three hybrid system as described by Liberies, S.D., *et al., Proc. Natl. Acad. Sci. USA* (1997) 94:7825-7830 and by Licitra, E.J., *et al*., *Proc. Natl. Acad. Sci. USA* (1996) 93:12817-12821; and the use of miniaturized formats by You, A. J., *et al., Chem. Biol.* (1997) 4:969-975 and Stockwell, B.R., *et al., Chem. Biol.* (1999) 61-83.

Phage display is a widely used method for identifying proteins and the genes encoding them which bind to other proteins. The original system as disclosed, for example, in U.S. patents 5,096,815 and 5,198,346 utilized the filamentous phage M13 and required translocation of the cloned protein across the *E. coli* inner membrane before incorporation into the phage particle. More recently, lytic bacteriophage vectors such as λ, T4, and T7 have been used, since they are independent of *E. coli* secretion. T7 is commercially available from Novagen and a detailed description of the use of T7 phage is provided in the company's T7 Select® System Manual and is described in U.S. patents 5,223,409; 5,403,484; 5,571,698; and 5,766,905, all incorporated herein by reference.

Although phage display systems have been successfully used to identify protein-protein interactions, it has not been reported to employ this system to identify proteins which bind to small molecule ligands. It is unclear why it is difficult for small molecule pharmaceuticals to select displayed proteins from a library, but this appears to be the case. Only recently, has it even been possible for small peptides to achieve this. For example, Zozulya, S., *et al*., *Nature Biotechnol* (1999) 17:1193-1198 describe the use of the T7 phage display system to map protein interactions linking epidermal growth factor receptor to the *Ras* protein. Various phosphorylated protein fragments were used as bait and were immobilized on streptavidin-coated paramagnetic beads.

Applicants are aware of only two papers describing the use of small peptides as bait; the paper by Zozulya, *et al*., set forth above and Cochrane, D., *et al., J. Mol. Biol*. (2000) 297:889-97.

The present invention extends the use of phage display to the interaction of cDNA libraries using traditional pharmaceutical-type small molecules as bait. This extension permits the direct identification of gene functionality by identifying phage which comprise nucleotide sequences encoding proteins that bind non-peptide small molecule ligands which are of known physiological function.

In addition to small molecule ligands whose physiological function is already known, compounds of unknown function can be used to ascertain their protein targets. Such molecules may be natural products, for example, or may be members of a combinatorial library.

### Disclosure of the Invention

The invention method provides a direct approach to identify proteins and genes encoding them, which proteins interact with a specific small molecule ligand. By use of the method of the invention, in which a multiplicity of proteins is displayed on bacteriophage, the phage containing nucleotide sequences that encode proteins capable of interacting with a specific small molecule bait can be "pulled out" of, for example, a cDNA library and identified. The successful phage can be amplified and sequenced, thus identifying the gene encoding the interacting protein and providing the ability to deduce the amino acid sequence of the protein.

Thus, in one aspect, the invention is directed to a method to identify a nucleotide sequence encoding a protein that binds a non-peptide target which method comprises amplifying and sequencing the nucleotide sequence contained in a bacteriophage, wherein the phage displays a protein which binds to the non-peptide bait. The phage is identified by a method which comprises contacting a phage display library comprising a multiplicity of DNA fragments encoding candidate proteins for said binding with said non-peptide bait, the non-peptide bait is coupled to a solid support under conditions wherein said non-peptide bait interacts with a displayed protein to which it binds specifically; and, if necessary, washing said solid support to remove non-specifically bound phage.

The specifically bound phage is then removed by elution and amplified and the DNA insert encoding the displayed protein sequenced.

In still another aspect, the invention is directed to a method to identify a compound which binds a protein identified by the above-recited method of the invention which method comprises contacting candidate compounds with the protein thus identified and assessing the ability of the compounds to interact with said protein.

In still another aspect, the invention is directed to compounds so identified and to methods of treating conditions characterized by the need to modulate the activity of the protein by administering the identified compound.

### Brief Description of the Drawings

Figure 1 shows a diagram of the method of the invention with one round of amplification.
Figure 2 shows, schematically, the synthesis of biotinylated FK506.
Figure 3 is a schematic representation of the T7 display vector and a diagram of the modified T7 gene.
Figures 4A and 4B show a model combinatorial compound and a schematic for the synthesis of a benzyl cycloadduct as a scaffold supported bait.
Figure 5 shows the coupling of the benzyl cycloadduct to an affinity support.

### Modes of Carrying Out the Invention

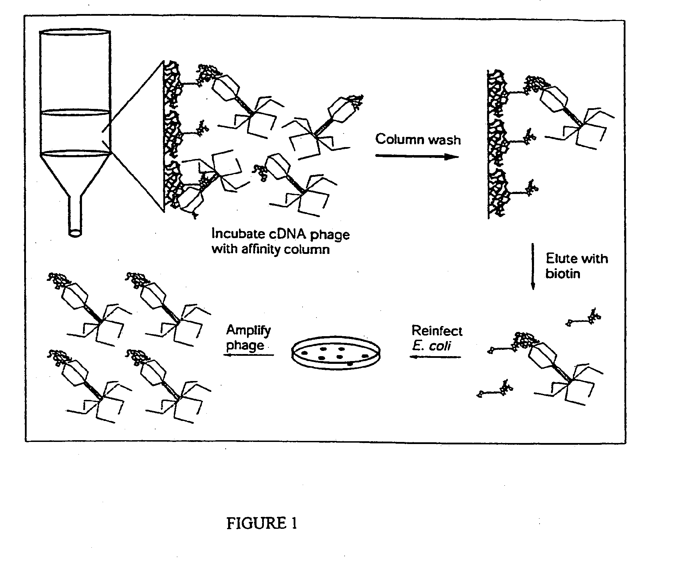

The invention generally employs a phage display system to provide an exposed protein for assay of the interaction with a small molecule. The small molecule is coupled to a solid support, preferably, but not necessarily, through a coupling system that is subject to elution using reagents which are independent of the nature of the small molecule. The successfully interacting phage are thus selectively bound to the solid support and after washing the support if necessary to remove un-bound phage, can be eluted and recovered for amplification and sequencing. In general, phage display systems involve incorporating genes encoding non-viral proteins into the viral genome so as to result in the production of fusion proteins between the desired foreign proteins and a coat protein of the virus. Thus, when the virus replicates, the foreign protein is displayed at its surface. Viral replication occurs in host cells. In a typical phage display library, a multiplicity of DNA-encoding foreign proteins is inserted and thus viral particles displaying a multiplicity of proteins is produced. This library of displayed proteins is then contacted with "bait" typically bound to a solid support. The successfully interacting displayed proteins will then preferentially be retained on the solid support and the non-binding proteins washed away. The bound protein-displaying phage are then eluted and amplified by culturing them in cells to produce a library enriched in proteins which bind the bait. Repeated rounds of amplification and selection may be required to obtain a relatively homogeneous population of phage which display only the protein or proteins which bind with high affinity to the bait. The nucleotide sequences contained in the genome of these phage can then be sequenced and the amino acid sequence of the binding protein deduced. A diagram showing, in general, the process described above is shown in Figure 1.

In previous work, successful isolation of phage which bind a defined bait has been possible only when the bait is itself a protein. This has limited the applicability of the technique; it has, for example, been used frequently to provide antibodies which bind to target antigens. However, the present applicants have succeeded in applying this technique to the identification of genes encoding proteins which bind small molecules. This has been demonstrated as described below by the isolation of the gene encoding FK506 binding protein (FKBP12) from a human brain cDNA library using FK506 as the bait. In addition, this technique has proved successful with respect to members of a combinatorial library. Thus, an arbitrary member of a combinatorial library is coupled to the solid support and is able to "pull out" a binding target protein. The protein thus obtained may have a known function, or may constitute a new potential target for molecules that are similar to the molecule which exhibits specific binding to the retrieved protein.

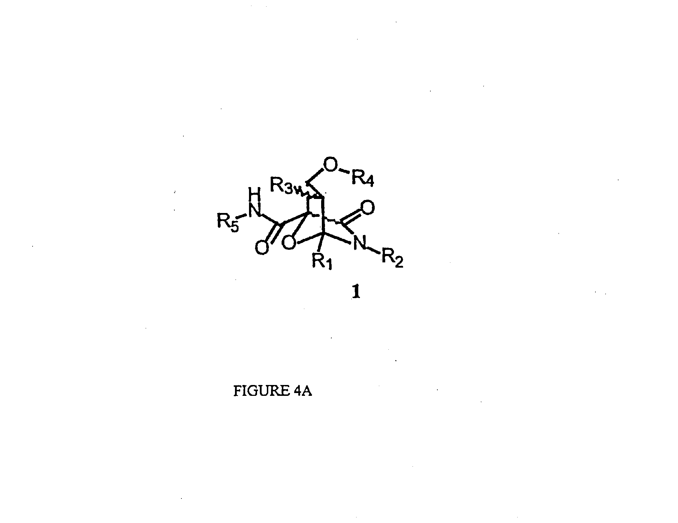

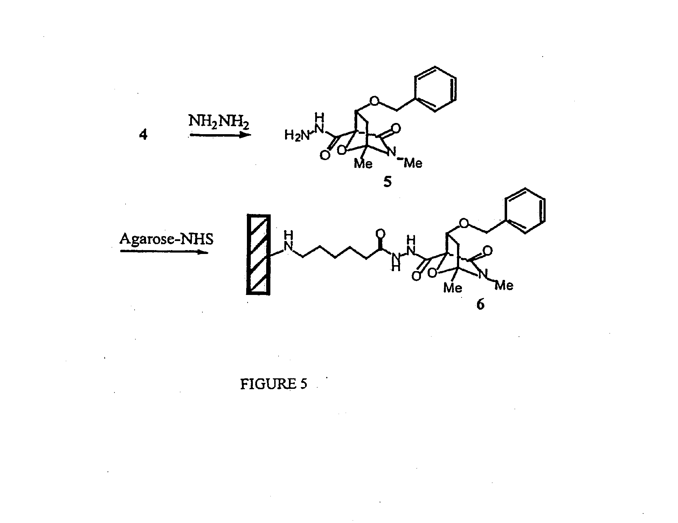

A particularly useful combinatorial library is disclosed herein. The library is based on a scaffold shown in formula 1 of Figure 4A where the defined portions of the molecule comprise the scaffold and R₁-R₅ represent variable substituents which can be manipulated at will. The scaffold containing the desired substituents can conveniently be coupled to a solid support through a linker such as that shown in Figure 5. In this case, the coupling of the bait to the solid support is covalent and the elution of the specifically bound phage comprises the use of a solution containing a compound which mimics the bait and thus displaces the coupled specifically bound phage or a change in solvent conditions. A successful experiment resulting in retrieval of a compound which binds a benzyl cycloadduct of formula 1 is set forth below in Example 2.

In general, the method of the invention relies on a phage display system wherein the inserted nucleotide sequences are typically members of a cDNA library. cDNA libraries can be assembled from a variety of sources, including single tissues such as brain, liver, stomach, prostate tissue, breast tissue, and the like from a variety of mammals or can be derived from any source, including yeast, invertebrates, plants, or prokaryotes. Libraries of the greatest interest are derived from mammals, particularly humans. The tissue may be derived from an organism which is considered "normal," or the organism or tissue may exhibit an abnormal condition, such as inflammation, tumor growth, hypertrophy, and the like. Techniques for preparing cDNA libraries are well known and such libraries can be obtained from very small samples, including single cells. Cell cultures may also be used. Various cDNA libraries are also available commercially.

The phage system can be selected from a variety of well known display systems, including the classical M13 system as well as λ phage, T4 and T7. Particularly preferred are the class of lytic phage, and in particular those in which the inserted nucleotide sequence tails the nucleotide sequence encoding a coat protein. The advantage of such tailed inserts is that the presence of a stop codon in the inserted nucleotide sequence does not prevent display of the encoded amino acid sequence as a contiguous fusion. The advantage of lytic phage resides in the ease with which the displayed protein can contact a selection means. One disadvantage of the M13 systems is that the phage containing the display protein must be secreted from *E. coli* in order to contact the selection system, thus conferring an advantage on normally secreted proteins, for example. However, lytic phage, because the host cell is lysed, can be immediately contacted with, for example, an affinity support.

In order for the method to be successful, the target protein must be displayed on the phage at a sufficient level that it can successfully be retrieved by the small molecule bait. Clearly, the smaller the number of different proteins displayed in the phage suspension (assuming that the protein which binds the bait is indeed included in the mixture) the higher the probability that the bait will capture the appropriate phage. Preferably, therefore, the phage display library will contain no more than 10⁷ different displayed proteins, preferably no more than 10⁶ different displayed proteins, more preferably no more than 10⁵ different displayed proteins, and most preferably no more than 10⁴ different displayed proteins. As described below, the number of different proteins displayed in the library is successively decreased with successive rounds of amplification; if the diversity of the library is reduced (without deleting the desired protein) the number of rounds of selection can be reduced as well.

According to the method of the invention, the selecting "bait" is a small molecule in the classical sense of that term. Thus, the molecule includes, for example, pharmaceuticals which are natural products or which are prepared synthetically, but which are not peptides or proteins. Thus, the non-peptide bait includes polyketides such as FK506 and erythromycin, synthetic drugs such as cefaclor, steroids such as finasteride, and the multiplicity of non-peptide molecules found in the *U.S. Pharmacopoeia,* the contents of which are incorporated herein by reference, to supply a non-limiting list of small molecules included within the scope of the invention.

As set forth above, the small molecule bait need not be a pharmaceutical or natural product of known function, but can be a new compound or an arbitrarily chosen compound whose function in biological systems, if any, is not known. A particularly useful source of such molecules is a combinatorial library wherein a multiplicity of compounds on a similar scaffold is constructed.

The small molecule bait is used to select a phage bearing a nucleotide sequence which encodes a protein that interacts with the bait by contacting a suspension of phage particles with a solid support to which the bait is coupled. The solid support can be any of a multiplicity of such supports, including agarose, polystyrene or other polyvinyl compounds, magnetic beads, and the like. The bait may be coupled covalently to the support or may be noncovalently bound by a system which permits the release of the entire complex between the bound phage and bait. For example, a column derivatized with N-hydroxysuccinimide can be used to couple covalently a carboxylic acid function on the bait molecule. For such coupling, elution of the bound phage would rely on competition with excess bait or other change of conditions. However, advantageously mild elution conditions may be used when the bait is coupled covalently only to a linker ligand which is itself noncovalently coupled to the support. Under those circumstances, the complex can be eluted in a manner not specific to the bait - for example, by supplying an excess of the linker. Illustrated below is a system wherein the bait is covalently bound to biotin which serves as a linker to noncovalently adsorb to an avidin derivatized column. After washing with buffer to remove non-bound phage, the phage/bait complex can be removed by treating the column with excess biotin. Similarly, the bait may be covalently coupled to other linkers such as polyhistidine which noncovalently associates with nickel chelates permitting removal or elution using excess polyhistidine, or can be coupled to glutathione which associates with a glutathione-S-transferase system coupled to the support or *vice versa*. A large number of such linkers which can be covalently bound to bait but noncovalently bound to a derivatized solid support is known, thus permitting a variety of non bait-specific elution protocols.

While this approach permits particularly mild elution conditions and may be advantageous, it is not a necessary feature of the invention. Covalent bonding of the bait to the solid support is also practical and elution can be effected by methods appropriate to this system.

A first round selection occurs when the phage display library containing the cDNA nucleotide sequences is contacted with the affinity support coupled to the non-peptide, small molecule bait, washed if necessary with, for example, buffer to remove non-specifically bound phage, and then eluted according to a procedure based on the mechanism used for coupling the bait to the affinity support. The eluted and thus selected phage members of the library can then conveniently be assessed for homogeneity by, for example, size separation of the cDNA inserts. If desired, the selected phage from the first round can be amplified and sequenced using standard techniques. Additional rounds of selection involve the same steps of contact with affinity support labeled with bait, wash, elution, and analysis, including, if desired, amplification and sequencing.

The number of rounds of selection desirable will depend on the purpose of the experiment. As shown below, only two rounds of selection were required to obtain an apparently homogeneous population of a binding protein with a high affinity for the FK506 bait. More weakly bound proteins may be obtained by analyzing the results of the first round of selection. On the other hand, if only weakly binding proteins exist in the library, several rounds of selection may be necessary to retrieve them with sufficient probability.

Retrieval of a phage which binds specifically to a small molecule ligand is useful in many contexts. First, it provides a direct method of revealing the cellular targets of small molecules, including natural products. This permits classification of proteins into groups as well as the identification of interactions that may have previously been unknown with respect to a pharmaceutical, thus revealing the nature of any side effects exhibited by administering the drug. Identification of the molecular targets involved in these side effects permits the design of formulations and co-administered drugs to alleviate such effects. Identification of additional targets for a pharmaceutical or other compound, such as a member of a combinatorial library, may reveal desirable interactions of these molecules with target proteins as well.

Once the protein that is the target of a small molecule non-peptide compound is identified, alternative small molecules which bind to the same target and have similar effects, but perhaps fewer side effects, for example, can readily be found. Thus, the identified protein may be used in screening assays for identification of compounds which also interact with it. Combinatorial libraries can readily be screened against such proteins, as well as can individual compounds. This permits identification of successful potential therapeutics which can also be assessed using the same methods for their side effects or lack thereof. For example, identification of the receptor which interacts with a pharmaceutical known to reduce inflammation would permit screening for other anti-inflammatory drugs. By also screening with respect to proteins which are responsible for side effects of the anti-inflammatory activity and comparing the ability of the test compounds to interact with these as compared to the known drug, compounds which inhibit inflammation but which exhibit fewer side effects can be identified. These compounds can then be used to treat inflammation in lieu of the previously used compound. Thus, the invention is directed to compounds identified by these screening assays and to methods to treat conditions mediated by the target proteins identified by the method of the invention.

The following examples are intended to illustrate but not to limit the present invention.

### Preparation A

### Synthesis of FK506-biotin (4)

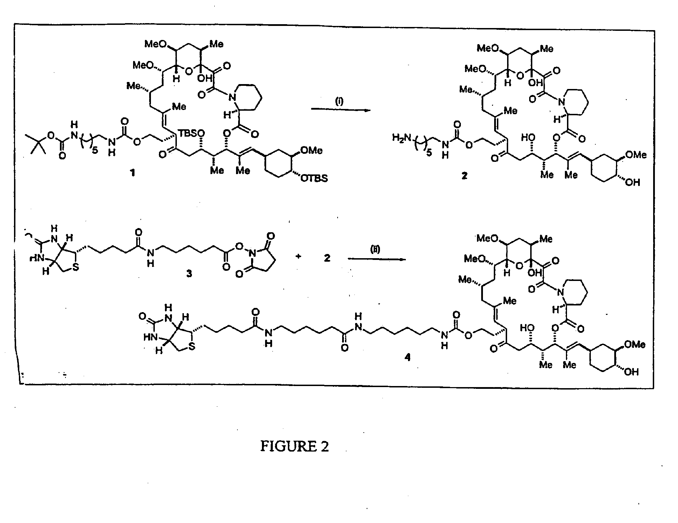

This synthesis is shown diagrammatically in Figure 2.

To a solution of Boc-FK506-TBS ₂ (7.5 mg, 5.8 mmol) [Belshaw, P.J., *et al*., *Proc. Natl. Acad. Sci. USA* (1996) 93:4604-4607] in 250 ml 7:1 CH ₃ CN:H ₂O was added 50 ml 48% HF in an Eppendorf tube. The solution was allowed to stir at room temperature until all of the starting material was consumed and ninhydrin staining revealed the presence of an amine with low R _{f} (0.1 in 31.1 ml t-butylacetate, 15.7 ml glacial acetic acid, 6.0 ml n-butanol and 14.4 ml water). Upon addition of NHS-LC-biotin (3.3 mg, 5.8 mmol; Pierce Chemical Company, Rockford, IL) the ninhydrin spot immediately disappeared and a new spot with a slightly higher R _{f} appeared. The reaction was quenched with saturated sodium bicarbonate (250 ml), extracted with methylene chloride (3 x 500 ml) and dried over sodium sulfate. The crude mixture was subjected to column chromatography (from 9:1 to 4:1, CH ₂ Cl ₂ :MeOH) and provided 1.7 mg (5.8 mmol) of FK506-biotin (4) (R _{f} 0.1 in 9:1 CH ₂ Cl ₂ :MeOH) in 35% yield.

### Preparation B

### Construction of human brain cDNA phage library

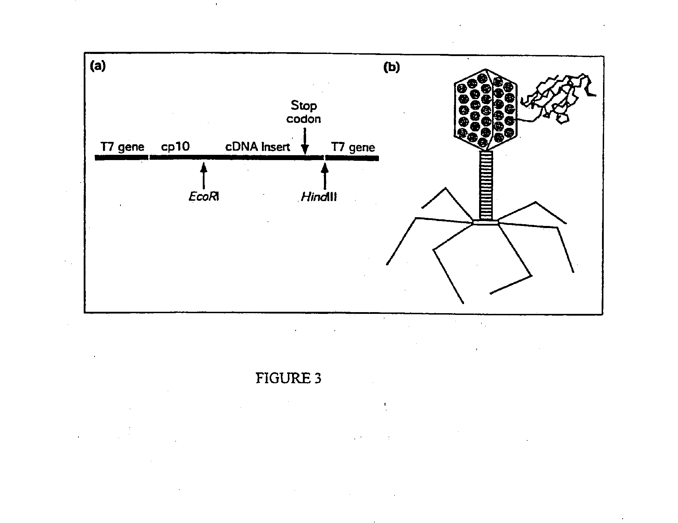

Bovine brain has traditionally been a good tissue source for the FKBP protein [Harding, M.W., *et al*., *Nature* (1989) 341:758-760]. As display cloning only requires a small amount of mRNA for library construction, human brain was chosen as the mRNA source. Purified human brain poly A⁺ mRNA (obtained from normal whole cerebral brain tissue of a 50 year old Caucasian male, Clontech, Inc., Palo Alto, CA) was randomly primed in the first-strand synthesis followed by second-strand synthesis to yield double-stranded complimentary DNA. The cDNA was doubly digested with EcoRI and HindIII endonucleases and size fractionated. The purified cDNA was then directionally ligated into T7 Select1-1b vector arms and subsequently *in vitro* packaged into phagemids (Novagen, Madison, WI). A diagram of the manner in which the DNA segments are inserted is shown in Figure 3. As shown, in the T7 System, the insert is downstream of the code protein (CP10) so the presence of a stop codon in the cDNA transcript does not destroy the ability of the phage to display the protein. Ligation efficiencies were evaluated with a small aliquot of the packaged phage and indicated a total of 3.3 x 10⁶ plaque forming units. These initial transformants were subjected to one amplification step by infecting 50 ml log phase BLT5403 cells, providing a human brain library of cDNA phage with an overall titer of 3.6 x 10¹⁰ pfu/ml. The library was stored in aliquots (100 ml) at -78°C as 8% glycerol stocks.

### Example 1

### Retrieval of cDNA Encoding FKBP12

A. To one liter of 2 x YT culture was added BLT5403 cells (from a 1 ml saturated culture) and incubated at 37°C until cell growth reached log phase (OD ₆₀₀ = 0.5-0.8). The culture was then inoculated with 10 ml (9 x 10⁸ phage) of the human brain cDNA library stock described above. The number of phage used was 200-fold over the original library diversity in order to maintain an appropriate representation of all library members. It is estimated that there are 2.0 x 10⁸ cells/ml in a culture at OD ₆₀₀ = 0.5. The level of infectivity (LOI) is kept above 1000 (LOI = # cells/# of phage) in order to ensure complete infection. This is important since a liquid culture amplification was performed. The culture was incubated at 37°C until completely lysed. To achieve high-salt conditions of the phage supernatant, 100 ml of 5 M NaCl was added and the suspension centrifuged at 4700 rpm for 20 minutes at 4°C in order to precipitate the cell debris. This supernatant solution had a final library titer of 1.8 x 10¹⁰ pfu/ml and was used directly in the affinity selections described below. By inoculating 1 liter of culture with about 10⁷ phage, a total of 10¹³ phagemids are formed. As the cDNA library contains 10⁶ distinct transformants, each clone is represented by approximately 10⁷ phage particles.
B. A bio-affinity column was prepared based on monomeric avidin bound to agarose, which has a relatively low affinity for biotin (K_{d} approximately 10⁻⁷ mol). This permits selective release of any biotin-bound species. The elution is thus independent of the FK506-protein interaction because the elution depends on competition of biotin for the entire complex containing biotinylated FK506 and phage.
   Three columns were prepared. One is the monomeric avidin column *per se,* the second, a biotin pre-treated column, and the third column treated with FK506 biotin.
   In more detail: Three one ml aliquots of monomeric avidin resin (ImmunoPure Immobilized Monomeric Avidin Gel, Pierce Chemical Company, Rockford, IL) were incubated with either (a) phage wash buffer (PWB) which is 1 x phosphate buffered saline, 450 mM NaCl, 0.05% Tween-20; or (b) 5 mM biotin in PWB, or (c) FK506-biotin (4) with slow rotation for 30 minutes at 4°C. All three columns were then rinsed with PWB (30 ml).
   In first round selection, 100 ml which contained about 10¹² phage were poured over each column. Thus, each member of the cDNA library is represented by approximately 10⁵ phage. The cDNA phage supernatant (100 ml) was poured over each column, followed by washing with PWB (100 ml) and distilled water (10 ml). The elution step for each column was identical and involved treatment with 10 ml of 5 mM biotin and collecting the eluate in 0.5 ml aliquots. A small amount (10 ml) from each fraction was used for titer determination.
   The initial fractions from the avidin column contained a phage titer of approximately 40 x 10⁴ pfu as compared to only 5 x 10⁴ pfu in the initial fractions of the biotin pretreated column. The titers rapidly declined in subsequent fractions of the avidin column. On the other hand, the FK506 biotin treated column showed an elevated titer relative to the biotin control column in later fractions.
   The fractions from the FK506 selection were combined and used to inoculate 100 ml of log phase BLT5403 cells and incubated at 37°C until completely lysed. After lysis, 10 ml of 5 M NaCI was added and the solution centrifuged at 4700 rpm for 20 minutes at 4°C to precipitate the cell debris. The supernatant contains the amplified phage from the first selection, however, it still has approximately 0.5 mM biotin from the previous elution. The biotin was removed through precipitation of the phage by adding 80 ml phage precipitant (33% PEG-8000, 3.3 M NaCI) and centrifuging at 7000 rpm for 10 minutes at 4°C. The precipitated phage were resuspended in 30 ml PWB, and a small amount (5 ml) was stored at -78°C as an 8% glycerol stock. An FK506-biotin affinity column was prepared as described above and the remaining 25 ml of the amplified phage were poured over the column. The column was washed with PWB (100 ml) and distilled water (10 ml), then eluted with 5 mM biotin (10 ml). The eluate for both the first and second selection rounds were plated onto 2 x YT top agar and single plaques were selected for DNA analysis.
C. Individual plaques, twenty prior to selection and five from each round of selection, were stabbed and used to inoculate log phase cultures of BLT5403 cells (10 ml) and allowed to incubate at 37°C until the culture completely lysed. Genomic DNA was isolated from the phage supernatants using standard lambda DNA purification resin (Wizard Lambda Preps DNA Purification System, Promega Corp., Madison, WI). Two primers, T7-up (5'-GGAGCTGTCGTATTCCAGTC-3') and T7-down (5'-AACCCCTCAAGACCCGTTTA-3') anneal to the vector sequence immediately flanking the cloning site and were used with standard PCR conditions to evaluate the relative size of the insert region of the isolated clones.

As all of the clones from the second round of selection appeared to have the same insert size, they were subjected to DNA sequencing and were subsequently found to be identical clones. Sequencing reactions were performed with the T7-up primer using fluorescently labeled dideoxynucleotides and Taq FS DNA polymerase in a thermal cycling protocol, and were analyzed on an Applied Biosystems 377 DNA Sequencer.

The amplified sequences contain 118 base pairs more than the gene insert, but the complete sequence of the gene insert was obtained. All of the clones from the second selection included the gene encoding the known FK506 binding protein, FKBP12 in its expressible form. The 5' and 3' untranslated regions and the start and stop codons were contained in the insert between the EcoRI and HindIII cloning sites. The clone obtained had the highest affinity (K_{d} = 0.4 nM) protein in the FKBP family of homologues. Thus, the procedure was successful, in only two rounds of selection, in isolating clones containing the cognate protein for FK506.

The assays performed above on the cDNA library cloned into T7 phage and after the first round of selection showed progressive homogeneity. There were no redundant clones in the original library and no duplicate sequences found after the first round of selection. One of the clones identified in the first round was that of the gene encoding FKBP12 which, as stated above, was the only clone recovered in the second round, out of those evaluated.

### Preparation C

### Synthesis of a benzyl cycloadduct

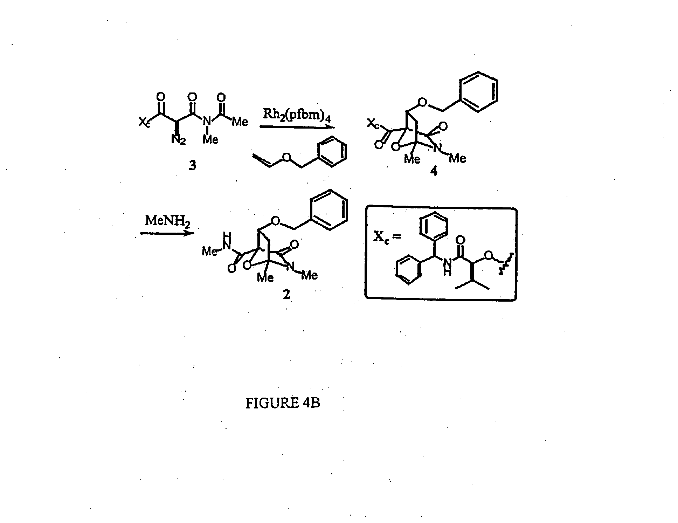

The synthesis of the small molecule bait which is a benzyl cycloadduct is outlined in Figure 4B. The diazoimide (3) was prepared by the procedure of Savinov, S.N., *et al., Chem. Commun.* (Cambridge) (1999) 1813-1814. Treatment of diazoimide (3) with rhodium(II) perfluorobutyramidate in methylene chloride at room temperature in the presence of benzyl vinyl ether yielded (4) in good yield (88%) and high diastereoselectivitiy (>98%).

The benzyl cycloadduct was then coupled to an agarose support which had been activated with an N-hydroxy succinimide and linked with aminocaproic acid as outlined in Figure 5. Treatment of (4) with hydrazine yielded hydrazide (5). The N-hydroxy succinimide (NHS) activated aminocaproic acid linked agarose support (3.3 mg, 0.2 mM) was treated with hydrazide (5) (20 ml, 100 mM, 50% DMSO/water) to provide benzyl scaffold agarose (6).

### Example 2

### Selection of Cognate Protein Binding Benzyl Cycloadduct

The human brain cDNA library described in Example 1 was used to inoculate log phase BLT5615 cells (Novagen, Inc.), containing 1 mM IPTG. The culture was incubated at 37°C until lysis was observed. The lysate was immediately treated with a protease cocktail (1% v/v 8.6 mg Sigma P8465 in 200 ml 20% DMSO/water) and adjusted to 10 mM DTT, 1% BSA, and 0.5 M NaCl. The solution was centrifuged at 14000 rpm for 15 minutes at 4°C to remove cell debris.

A 1 ml aliquot of the freshly prepared phage lysate was allowed to batch incubate with the probe support (6) (10 ml) for 30 minutes. The support was column washed with 1 ml of phage wash buffer (PWB) (0.05% Tween-20 in PBS) followed by batch incubation with 500 ml PWB for 5 minutes. The bound phage were batch eluted by treating the support with 100 ml of a 10 mM solution of amide (2) in Luna Broth containing 1% BSA for 5 minutes.

Six clones from the first round were randomly selected and analyzed by polymerase chain reaction (PCR) amplification and subsequent DNA sequencing. None of the first round clones contained an open reading frame (ORF).

The overall first round phage titer for the specific elution with scaffold 2 was 6x10⁵ pfu, (plaque forming units) while the titer of the last wash was 7x10⁵ pfu. The lack of significant titer increase was due to the wash step using a small amount of detergent (0.01% Tween-20), which contributes to a higher titer in the wash solution.

A second round of affinity selection was performed to allow the amplification of any binding proteins of lower affinity that might require more than one round of selection in order to be detected. The second round specific elution titer increased to 33x10⁵ pfu, while the last wash titer remained low (6x10⁵ pfu), indicating a potential phage enrichment.

Of four clones analyzed, one clone was found to contain the entire coding region of the α domain of F₁ ATP synthase, an ATP binding protein. Although the amount of background binding observed in the first round was high, the second selection round increased the signal, as exemplified by the presence of the F₁ ATP synthase gene.

A confirmatory experiment was performed to validate the ability of the α domain of F₁ ATP synthase to bind the benzyl cycloadduct. In a manner similar to that set forth above, two phage systems were mixed in a 1:1 ratio - a phage containing no insert and expressing no surface protein and a phage expressing the F₁ α ATP synthase at its surface. The mixture was subjected to one round of selection as described above and assayed, resulting in an approximate two-fold increase in the F₁ α ATP phage as compared to control.

## Claims

1. A method to isolate a protein or its encoding nucleotide sequence wherein said protein binds specifically to a non-peptide bait which method comprises
contacting a phage display library comprising a multiplicity of different DNA fragments encoding candidate proteins for said binding with said non-peptide bait, wherein said non-peptide bait is coupled to a solid support under conditions wherein said non-peptide bait interacts with a displayed protein to which it binds specifically; and
optionally washing said solid support to remove non-specifically bound phage.

2. The method of claim 1 which further includes eluting said specifically bound phage from the solid support.

3. The method of claim 1 wherein said phage display library is constructed from a lytic bacteriophage vector.

4. The method of claim 3 wherein the lytic bacteriophage vector is T7.

5. The method of claim 1 wherein said non-peptide bait is non-covalently bound to the solid support.

6. The method of claim 5 wherein said non-covalent binding is effected by biotinylated bait coupled to monomeric avidin, wherein said monomeric avidin is covalently bound to the solid support.

7. The method of claim 1 wherein said multiplicity of DNA fragments comprises a cDNA library constructed from a specialized tissue or cellular source.

8. The method of claim 7 wherein said specialized tissue is brain.

9. The method of claim 1 wherein said multiplicity of DNA fragments is less than 10⁵ different fragments.

10. A method to identify a nucleotide sequence encoding a protein that binds a non-peptide bait which method comprises amplifying and sequencing a nucleotide sequence contained in a bacteriophage displaying a protein which binds to a non-peptide bait, wherein said phage is identified by the method of claim 1.

11. A method to produce a protein which binds specifically to a non-peptide bait which method comprises providing an expression system for the nucleotide sequence identified by the method of claim 10 to a recombinant system for expression of said nucleotide sequence and
effecting the expression of said nucleotide sequence to produce said protein.

12. A method to identify a compound which binds to a protein receptor, which method comprises contacting the protein produced by the method of claim 11 with a candidate compound; assessing the ability of said protein to interact with or bind said candidate compound; and
identifying as a successful candidate a compound which interacts with or binds said protein.

13. A successful candidate compound identified by the method of claim 12.

14. A method to treat a condition in a subject modulated by a receptor protein which method comprises administering to said subject the compound of claim 13.

15. A method to separate a compound which specifically interacts noncovalently with a cognate from a mixture of compounds which do not interact specifically with said cognate which method comprises providing a solid support to which said cognate is noncovalently coupled through an interaction between a first substance covalently linked to said cognate and a second substance which noncovalently binds the first substance;
applying said mixture of compounds to said solid support whereby a compound which interacts specifically with said cognate is retained by said solid support;
optionally washing said solid support to remove any nonspecifically coupled compounds in the mixture; and
treating said solid support with a solution containing a sufficient concentration of said first substance to elute from said support said compound associated with its cognate, wherein said cognate is a non-peptide small molecule and said compounds are phage displayed proteins.

16. The method of claim 15 wherein said first substance is biotin and said second substance is avidin.

17. The method of claim 15 wherein said first substance is polyhistidine and said second substance is a nickel chelate.

18. A method to isolate a protein or its encoding nucleotide sequence wherein said protein binds specifically to a non-peptide bait which method comprises eluting a phage which displays said protein from a solid support wherein said solid support is coupled to said non-peptide bait.

19. The method of claim 18 wherein said non-peptide bait is noncovalently bound to the solid support.

20. The method of claim 19 wherein said bait is covalently linked to a first substance which binds noncovalently to a second substance covalently linked to said solid support.

21. The method of claim 20 wherein the first substance is biotin and the second substance is avidin.
